# EUROPÄISCHE PATENTSCHRIFT

(11) **EP 0 812 305 B1**
(45) Veröffentlichungstag und Bekanntmachung des Hinweises auf die Patenterteilung: **02.09.1998**
(21) Anmeldenummer: 96904858.6
(22) Anmeldetag: 24.02.1996
(51) Int. Cl.: C03C 11/00, C03C 1/00, B01J 37/03

(54) **MIKROPORÖSE AMORPHE MISCHMETALLOXIDE FÜR FORMSELEKTIVE KATALYSE**
MICROPOROUS AMORPHOUS MIXED METAL OXIDES FOR FORM-SELECTIVE CATALYSIS
OXIDES METALLIQUES MELANGES AMORPHES MICROPOREUX POUR CATALYSE A SELECTION DE FORMES

(30) Priorität: 28.02.1995 DE 19506843
(43) Veröffentlichungstag der Anmeldung: 17.12.1997
(73) Patentinhaber: Studiengesellschaft Kohle mbH, 45470 Mülheim (DE)
(72) Erfinder: MAIER, Wilhelm, F. Studiengesellschaft Kohle mbH, D-45470 Mülheim (DE)
(74) Vertreter: von Kreisler, Alek, Dipl.-Chem.
(86) Internationale Anmeldenummer: EP9600766
(87) Internationale Veröffentlichungsnummer: WO9626907

(56) Entgegenhaltungen:
- EP-A- 0 590 714
- US-A- 4 765 818
- JOURNAL OF APPLIED PHYSICS, Bd. 61, Nr. 12, 15.Juni 1987, NEW YORK US, Seiten 5438-5446, XP002007175 M.W. SCHAFER ET AL.: "THE CHEMISTRY OF AND PHYSICS WITH POROUS SOL GEL GLASSES"
- JOURNAL OF NON-CRYSTALLINE SOLIDS, Bd. 147/148, Nr. 1, Oktober 1992, AMSTERDAM NL, Seiten 724-738, XP000398467 M.A. CAUQUI ET AL.: "APPLICATION OF THE SOL GEL METHODS TO CATALYST PREPARATION"
- CHEMICAL ABSTRACTS, vol. 104, no. 24, 16.Juni 1986 Columbus, Ohio, US; abstract no. 211888, Seite 283; XP002007176 & JP,A,06 117 443 (AGENCY OF INDUSTRIAL SCIENCES AND TECHNOLOGY) 25.Januar 1986
- CHEMICAL ABSTRACTS, vol. 115, no. 4, 29.Juli 1991 Columbus, Ohio, US; abstract no. 34181t, Seite 300; XP000214050 & JP,A,02 311 329 (MITSUBISHI KASEI CORP.) 26.Dezember 1990

## Beschreibung

Trotz des großen Fortschritts in der heterogenen Katalyse hat sich die katalytische Selektivität zu einem immer wichtigeren Prüfstein für technische Anwendungen von neuen heterogenen Katalysatoren entwickelt. In allen wichtigen chemischen Produktionsverfahren sind die technischen Selektivitätsgrenzen der derzeitigen Katalysatorengeneration höchstwahrscheinlich erreicht. Hoch entwickelte chemische Reaktionstechniken und Katalysatorverbesserungen durch semi-empirische Katalysatormodifizierung durch Additive, Oberflächenmodifizierung und Porenstrukturoptimierung sind Stand der Technik in der chemischen Prozeßentwicklung. Ein erster Schritt zur Substratselektivität, die Formselektivität, ist der Schlüssel zum Erfolg der Zeolithe als bedeutendste neue Generation heterogener Katalysatoren. Unter Formselektivität versteht man eine chemische Produktselektivität, die aufgrund der unterschiedlich eingeschränkten Beweglichkeit von den verschiedenen Produktmolekülen in den Poren des Katalysators zustande kommt. Voraussetzung ist, daß die Poren des Katalysators in ihrem Durchmesser nur geringfügig größer sind als die Produktmoleküle. So wird zum Beispiel mit dem sauren H-ZSM-5 Zeolith die Bildung von para-Xylol durch Isomerisierung von Xylolgemischen erzielt, da das paraXylol aufgrund seiner gestreckten Form in den engen Porenkanälen des Zeolithen wesentlich schneller diffundieren kann als das sperrigere ortho- und meta-Xylol (D.H. Olsen, W.O. Haag, ACS Symp. Ser. 248 (1984) 275). Die Formselektivität von Zeolithen in einer großen Zahl unterschiedlicher Reaktionen (P.B. Venuto, Microporous Materials 2 (1994) 297) wird auf deren mikroporöses Kanalsystem mit Porenabmessungen in der Größe von Molekülen zurückgeführt. Obwohl die Anzahl der Zeolithstrukturen und die dazu gehörigen Porengrößen kontinuierlich zunehmen, wird das genaue Maßschneidern von Zeolithen durch folgende Fakten eingeschränkt:
i) Die Porengrößenvariation ist nicht kontinuierlich, sondern abhängig vom verfügbaren Kristalltyp, und daher nur stufenweise möglich.
ii) Monomodale Porengrößenverteilungen mit Porendurchmessern im Bereich von 0,8 - 1,2 nm sind nicht bekannt.
iii) Die Konzentration und der Einbau zweier Elemente in der Zeolithgerüststruktur ist stark eingeschränkt.
Während nur sehr wenige Elemente wie Ti, Al, P, V, sich in der Silikatstruktur isomorph substituieren lassen, ist die mögliche Konzentration im günstigsten Fall bis 50 % erreicht und überschreitet häufig nicht einmal 2 - 3 %. Zeolithe werden bereits für eine große Zahl organischer Reaktionen als selektive heterogene Katalysatoren eingesetzt (P.B. Venuto, Microporous Materials 2 (1994) 297).

Wir haben nun gefunden, daß amorphe mikroporöse Mischmetalloxide mit einer extrem engen Mikroporenverteilung und Porendurchmessern im Bereich von 0,5-1 nm in ähnlicher Weise wie die kristallinen Zeolithe als formselektive Katalysatoren fungieren. Wir haben gefunden, daß mit solchen Katalysatoren t-Butylether direkt aus n-Alkoholen und t-Butylalkohol oder Isobuten hergestellt werden können. Unter homogenen Bedingungen wird keine t-Butyletherbildung beobachtet. Diese Materialien katalysieren auch die Bildung von Epoxiden durch direkte Oxidation von Olefinen mit 6 oder weniger Kohlenstoffatomen viel schneller als die Bildung der Epoxide größerer Alkene. Die Produktzusammensetzung beim hydrierenden Cracktest von Decan ist vergleichbar mit der Produktverteilung , die durch Katalyse mit großporigen Zeolithen, wie Y-Zeolithen, dem Zeolith Beta oder SAPO's entsteht. Wir haben gefunden, daß solche katalytisch aktiven amorphen mikroporösen Mischmetalloxide nach einem modifizierten Sol-Gel-Verfahren hergestellt werden können. Von besonderer Bedeutung für diese Herstellung ist, daß mindestens eine der Metallkomponenten, vorzugsweise ein Si, Ti, Al oder Zr-derivat, flüssig oder in Lösung vorliegen muß und daß die Polykondensation im Sol-Gel-Prozeß nicht unter basischen Bedingungen durchgeführt wird. Es werden also keine Membranen hergestellt, vielmehr wird das entstandene Gel schonend sofort getrocknet. Wir haben durch rheologische Untersuchungen gefunden, daß der Sol-Gel-Prozeß, katalysiert unter sauren bis neutralen Reaktionsbedingungen, mit einer linearen Polymerisation beginnt, so daß im entstehenden Gel die Viskosität zusammen mit der Elastizität zunimmt. Wird ein so erhaltenes Gel nun langsam getrocknet und mit kleinen Aufheizgeschwindigkeiten gebrannt, so entsteht ein mikroporöses Glas, in dem die verschiedenen Metalloxide atomar oder nahezu atomar miteinander vermischt sind, d. h. ohne Domänenbildung der einzelnen Metalloxide. Dieses mikroporöse Glas wird nun zur gewünschten Korngröße vermahlen und repräsentiert den zur formselektiven Katalyse einsetzbaren Katalysator.

Die Erfindung betrifft ein Verfahren zur Herstellung formselektiv katalytisch aktiver, amorpher, mikroporöser Mischmetalloxide nach dem Sol-Gel-Verfahren, das dadurch gekennzeichnet ist, daß mindestens zwei hydrolysierbare, flüssige oder gelöste Verbindungen der Elemente Titan, Silicium, Aluminium, Zirkon oder Cer hintereinander ineinander gelöst werden, die klare Lösung bei pH 0 bis 7 unter Zusatz wässriger saurer Katalysatoren oder unter Zusatz von Fluoridionen unter linearer Polymerisation oder Polykondensation gerührt werden, das erhaltene Gel durch Erwärmen auf 60 bis 70 °C schonend getrocknet wird und mit kleinen Aufheizgeschwindigkeiten bei Temperaturen von 120 bis 800 °C kalziniert wird, wobei ein midroporöses, amorphes Glas erhalten wird.

Die erhaltenen mikroporösen, amorphen (mit homogener Verteilung der Elemente, d.h. homogenes Glas (keine Partikel)), nicht-keramischen Gläser bestehen aus einer Mischmetalloxidmatrix, in der wenigstens etwa 90 % der Poren des Materials einen effektiven Durchmesser zwischen 0,3 und 1,2 nm haben, mit im wesentlichen gleicher Porengröße und mit einer Oberflächengröße von über 50 m²/g.

Die hydrolisierbaren flüssigen oder gelösten Verbindungen sind bevorzugt aus der Gruppe SiO₂, TiO₂, Al₂O₃, Zirkonoxid, Ceriumoxid, Spinell, Mullit, Siliciumcarbid, Siliciumnitrit und Titannitrit ausgewählt.

In einer weiteren Ausführungsform enthält die Mischmetalloxidmatrix wenigstens 50 Gew.-% wenigstens einer Verbindung der Elemente Titan, Silicium, Aluminium, Zirkon und Cer und bis zu 50 Gew.-% einer oder mehrerer Metalloxide in atomarer Verteilung aus der Gruppe der Metalle Mo, Sn, Zn, V, Mn, Fe, Co, Ni, As, Pb, Sb, Bi, Ru, Re, Cr, W, Nb, Hf, La, Ce, Gd, Ga, In, Tl, Ag, Cu, Li, K, Na, Be, Mg, Ca, Sr und Ba.

Weiterhin kann die Mischmetalloxidmatrix zusätzlich bis zu 5 Gew.-% wenigstens eines der Edelmetalle Pt, Rh, Ir, Os, Ag, Au, Cu, Ni, Pd, Co in hochdisperser Form in metallischem oder oxidiertem Zustand enthalten.

Bevorzugt sind als die sauren Katalysatoren Säuren, insbesondere Salzsäure.

Bevorzugt beträgt die Kalzinierungstemperatur 250 bis 500 °C.

In einer bevorzugten Ausführungsform sind die hydrolysierbaren löslichen Verbindungen reine Alkoxy-, gemischte Alkoxy-, Alkoxyoxo- oder Acetylacetonat-Derivate der gewählten Metalle oder Metalloxide.

Die Erfindung betrifft ferner mikroporöse, amorphe, nicht-keramische Gläser, bestehend aus einer Mischmetalloxidmatrix, in der wenigstens etwa 90 % der Poren des Materials einen effektiven Durchmesser zwischen 0,3 und 1,2 nm haben, mit im wesentlichen gleicher Porengröße und mit einer Oberflächengröße von über 50 m²/g.

Bevorzugt besteht die Mischmetalloxidmatrix aus wenigstens zwei der Oxide von Titan, Silicium, Aluminium, Zirkon oder Cer.

Insbesondere besteht die Mischmetalloxidmatrix aus wenigstens zwei der Verbindungen aus der Gruppe SiO₂, TiO₂, Al₂O₃, Zirconiumoxid, Ceriumoxid, Spinell, Mullit, Siliciumcarbid, Siliciumnitrid und Titannitrit.

In einer weiteren Ausführungsform besteht die Mischmetalloxidmatrix aus wenigstens 50 Gew.-% einer der Verbindungen der Elemente Titan, Silicium, Aluminium, Zirkon oder Cer und bis zu 50 Gew.-% einer oder mehrerer Metalloxide in atomarer Verteilung aus der Gruppe der Metalle Mo, Sn, Zn, V, Mn, Fe, Co, Ni, As, Pb, Sb, Bi, Ru, Re, Cr, W, Nb, Hf, La, Ce, Gd, Ga, In, Tl, Ag, Cu, Li, K, Na, Be, Mg, Ca, Sr, und Ba.

Weiterhin kann die Mischmetalloxidmatrix zusätzlich bis zu 5 Gew.-% wenigstens eines der Edelmetalle Pt, Rh, Ir, Os, Ag, Au, Cu, Ni, Pd, Co in hoch-disperser Form in metallischem oder oxidiertem Zustand enthalten.

Die mikroporösen amorphen nicht-keramischen Gläser sind erhältlich durch saure oder Fluorid-katalysierte lineare Polymerisation oder Polykondensation hydrolisierbarer, löslicher, oben genannter Verbindungen, vorzugsweise reinen Alkoxy-, gemischen Alkoxyalkyl-, Alkoxyoxo- oder Acetylacetonat-Derivate der gewählten Metalle oder Metalloxide bei pH 0 bis 7 in einem Sol-Gel-Verfahren, gefolgt von mildem Trocknen und langsamem Kalzinieren mit einer Endkalzinierungstemperatur im Bereich von 120 bis 800 °C, wobei die mikroporösen, amorphen, nicht-keramischen Gläser entweder ausschließlich aus den gemischten Metalloxiden oder aus den gemischten Metalloxiden und restlichen Oberflächenalkyl- oder -alkoxygruppen in Abhängigkeit von der gewählten Vorläufer-Verbindung bestehen.

Die Erfindung betrifft auch formselektive Katalysatoren, bestehend aus den vorstehend definierten mikroporösen, amorphen, nicht-keramischen Mischmetalloxidgläsern.

Schließlich betrifft die Erfindung die Verwendung der mikroporösen, amorphen, nicht-keramischen Mischmetalloxidgläsern bzw. der formselektiven Katalysatoren zur Katalyse von Isomerisierungsreaktionen, Hydrierreaktionen, selektiven und unselektiven Oxidationsreaktionen mit Luftsauerstoff, Wasserstoffperoxid oder organischen Peroxiden, Alkylierungsreaktionen, Disproportionierungsreaktionen, Hydrier- und Dihydrierreaktionen, Alkoholbildung aus Olefinen, Kupplungsreaktionen, Substitutionsreaktionen, Cycloadditions- oder Cycloreversionsreaktionen, Etherbildung, Rohölcracking und Hydrocracking, Fischer-Tropsch-Synthese von Alkoholen oder Kohlenwasserstoffen, Methanolsynthese aus Synthesegas oder aus Methan, zur Beschichtung von Elektroden in Brennstoffzellen oder Li+ oder anderen Ionenspeichern in Batterien, zur Bildung von Ultrafiltration- und Gastrennmembranen und zur Bildung von Katalysatoren mit selektiven Hohlräumen zur molekularen Erkennung.

Die so erhaltenen Mischmetalloxide unterscheiden sich schon in der Herstellung deutlich von der Herstellung mikroporöser Membranen (DE -A - 41 17 284), bei denen noch vor Erreichen des Gel-Punktes durch Herausziehen der Trägermembran aus der Sol-Lösung ein dünner, kontinuierlicher Film auf der Trägermembran gebildet wird, der sich durch den beschriebenen Trocknungs- und Kalzinierprozeß direkt in die mikroporöse Membran umwandelt. Auch die Anwendung der Membran unterscheidet sich grundsätzlich von der Anwendung der formselektiven Katalysatoren. Während die Membranen zur molekularen Trennung von Gas- und Flüssigkeitsgemischen genutzt werden, bei der eine der abzutrennenden Komponenten möglichst vollständig auf einer Seite der Membran verbleibt und die Membranen nicht oder kaum penetrieren kann, ist es für die formselektive Katalyse essentiell, daß alle Reaktanden das Porensystem des Katalysators penetrieren und in den Poren zur Reaktion kommen.

Die hier beschriebenen neuen Materialien unterscheiden sich ebenso wie die soeben beschrieben in der Herstellung von den mikroporösen Membrankatalysatoren (DE - A - 43 03 610). In der Anwendung unterscheiden sich die Membrankatalysatoren von den formselektiven Katalysatoren vor allem darin, daß die Katalyse mit Membrankatalysatoren auf der selektiven Trennung von Gas- und Flüssigphase in Dreiphasenreaktionen beruht und Verbesserungen gegenüber herkömmlicher heterogener Katalyse in dem molekülgrößenselektiven Ausschluß von unerwünschten Reaktionspartnern oder Katalysatorgiften beruht. In der Membrankatalyse werden Katalysator- und Membraneigenschaften gleichzeitig genutzt, während bei formselektiver Katalyse alle Reaktionspartner im Porensystem anwesend sein müssen. Im Unterschied zur Membrankatalyse können die hier beschriebenen Katalysatoren direkt als Pulver- oder Formkörper-Katalysatoren unter Verwendung herkömmlicher Reaktortechnologie zur selektiven heterogenen Katalyse eingesetzt werden. Während beim Einsatz von Membranen die Selektivität der Katalyse auf dem Ausschluß mindestens eines der Reaktanden aus dem Porensystem der Membran beruht, ist in den hier beschriebenen Katalysatoren die Anwesenheit aller Reaktanden in den Poren zwingend notwendig.

Die größte Ähnlichkeit mit den hier beschriebenen Materialen besitzen die mikroporösen Metalloxide (W.F. Maier, I.-C. Tilgner, M. Wiedorn, H.-C. Ko, Advanced Materials 5 (1993) 726). Diese Monometalloxide unterscheiden sich von den hier vorgestellten Materialien vor allem dadurch, daß die für die katalytische Aktivität verantwortlichen Mischmetallkomponenten als integraler Bestandteil der Glasmatrix fehlen. Es ist neu und bisher nicht bekannt, daß es möglich ist, Mischmetalloxide mit der hier beschriebenen, mit Zeolithen vergleichbaren engen Mikroporenverteilung und gleichzeitiger homogener Vermischung der Metalloxidkomponenten als thermisch und chemisch stabile Materialien herzustellen.

Die hier vorgestellten Materialien unterscheiden sich auch von den Katalysatoren zur selektiven Hohlraumkatalyse (DE - A - 43 09 660). Während die erfindungsgemäßen hier beschriebenen vorgestellten Materialien die selektive Katalyse durch die eingeschränkte Beweglichkeit der Moleküle im Inneren der Kanalstruktur verursachen, beruht die selektive Katalyse an molekularen Abdrücken auf der molekularen Erkennung bestimmter Strukturen. Selektive Hohlraumkatalysatoren müssen für eine ganz bestimmte Struktur maßgeschneidert werden und sind in ihrer Herstellung wesentlich aufwendiger als die jetzt gefundenen Materialien. Die Katalysatorherstellung unterscheidet sich vor allem darin, daß bei selektiven Hohlraumkatalysatoren eine Copolykondensation mit dem, an einer Monomereinheit über eine chemische Bindung verankerten Abdrucksmolekül zu erfolgen hat, welches vor dem Einsatz als Katalysator aus dem Glas enfernt werden muß. Diese Schritte entfallen bei den jetzt gefundenen Katalysatoren vollständig.

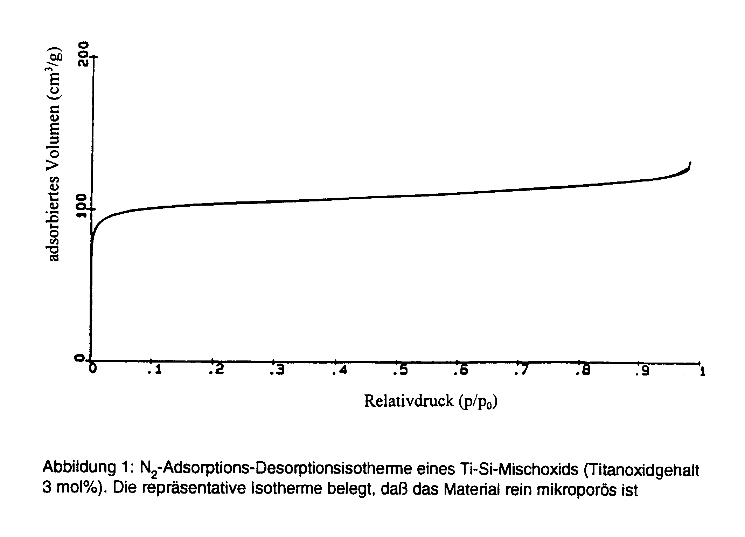

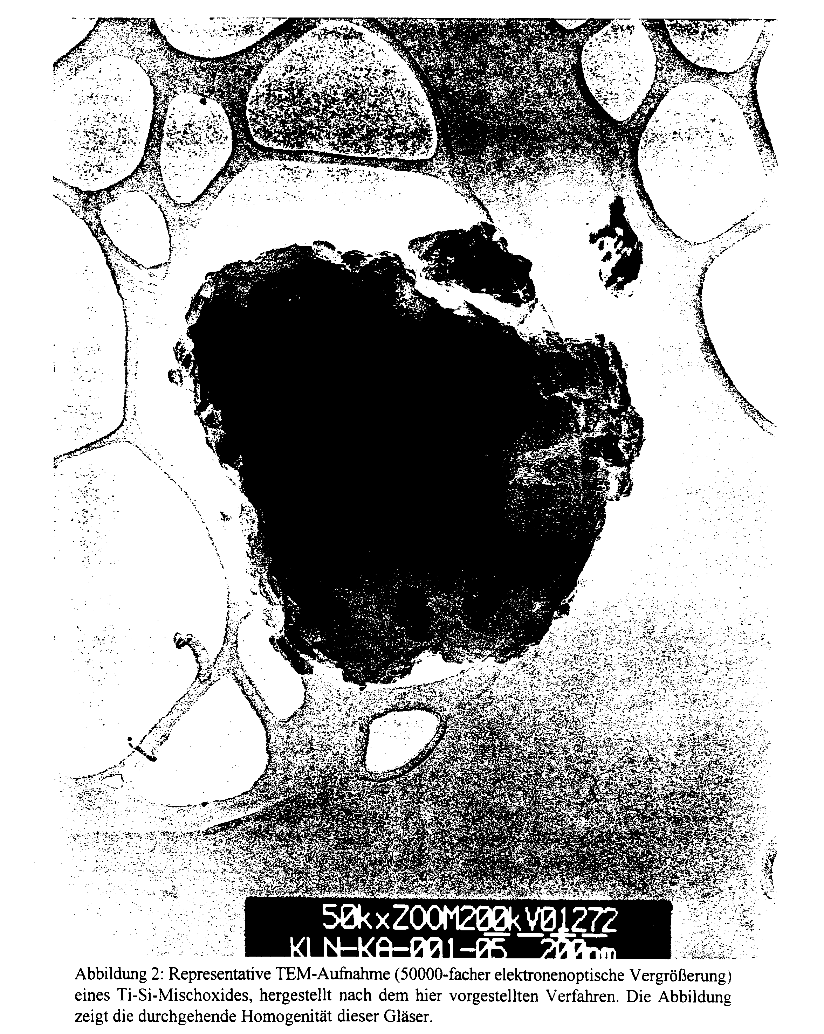

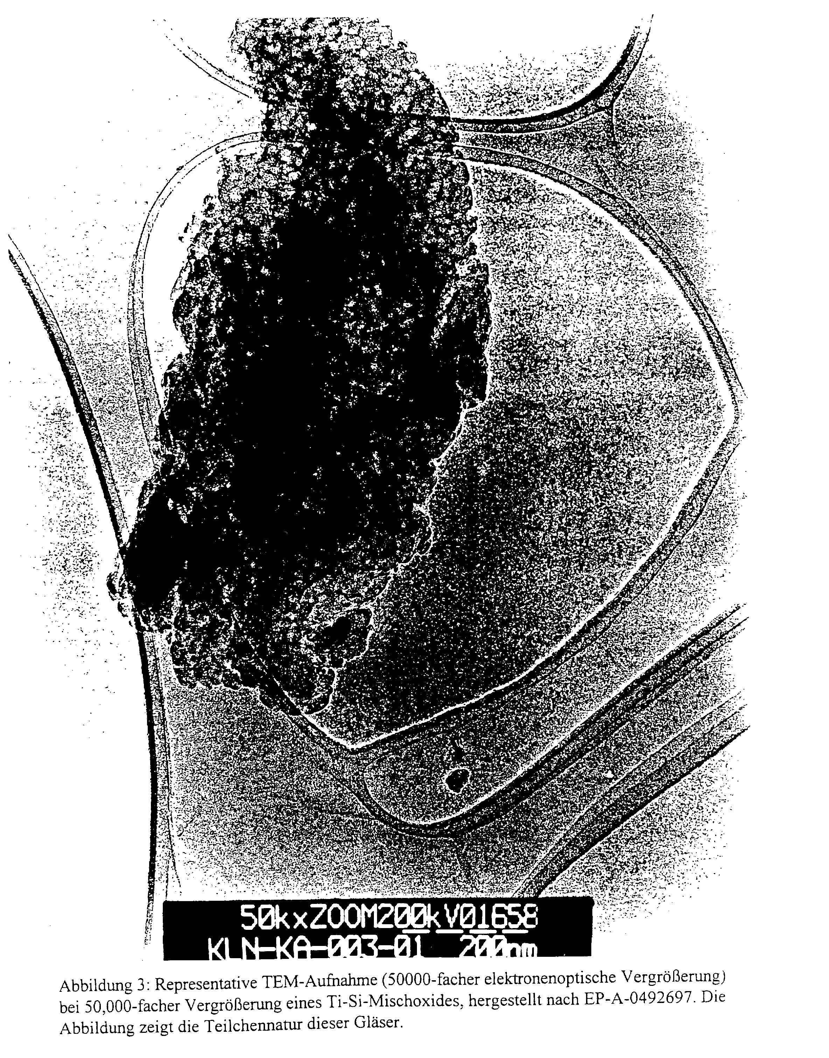

Die katalytische Aktivität von amorphen Mischmetalloxiden ist allgemein bekannt. In der EP - A - 0 492 697 wird zum Beispiel die Herstellung von Mischmetalloxiden aus Tetraalkylsilikaten und einer wasserlöslichen Form eines zweiten Metalles durch ein basisches Polykondensationsverfahren in der Gegenwart von Tetraalkylammoniumhydroxid beschrieben. In diesem Verfahren beginnt die Polymerisation durch die Bildung kleinster Teilchen und das so erhaltene Glas, obwohl mikroporös, zeigt signifikante Bildung von Mesoporen, die auf die Hohlräume zwischen den Glasteilchen zurückgeführt werden können. Beides, die Mesoporen und das Zwischenkornvolumen sind unerwünscht, wenn formselektive Katalyse erzielt werden soll. Wir haben nun gefunden, daß säurekatalysierte Polycokondensation von Tetraalkylsilikaten von bis zu 50 % eines löslichen zweiten Metallsalzes zur Herstellung mikroporöser Gläser ohne Mesoporenkontamination herangezogen werden kann. Abbildung 1 zeigt die typische N₂-Ad-Desorptions-Isotherme, in welcher keine Anzeichen einer zweiten Adsorption aufgrund von Mesoporen erkennbar ist. Die Isotherme eines solchen Materials ist identisch mit der Isotherme kristalliner Zeolithe.

Der Unterschied zwischen unseren Materialien und den Materialien, hergestellt nach EP - A - 0 492 697 ist, daß unsere Materialien im pH-Bereich 0 - 7 und ohne Template hergestellt werden. Unsere Materialien zeigen eine saubere monomodale Porengrößenverteilung in der Adsorptions- und Desorptionsisotherme, während ein Material, hergestellt nach EP - A - 0 492 697 einen deutlichen Anteil mesoporöser Flaschenhalsporen, erkennbar in der N₂-Desorptionsisotherme, aufweist. Bei atomarer Auflösung in der Durchdringungselektronenmikroskopie zeigt sich unser Material immer noch als homogenes Glas (Abb. 2 - 1272), während das Material nach EP - A - 0 492 697 sich aus verschmolzenen Teilchen (Abb. 3 - 1658) zusammensetzt, was darauf hinweist, daß zu Beginn die Polykondensation nach dem bekannten Verfahren von Teilchenwachstum dominiert wird, während unter erfindungsgemäßen Bedingungen die Materialbildung durch lineare Polymerisation dominiert. Bei der Bildung von t-Butylether aus Isobuten in n-Hexanol zeigt das als bekannt beschriebene Material unter unseren Standardbedingungen nur die halbe Aktivität unserer mikroporösen Gläser.

Die erfindungsgemäßen mikroporösen amorphen Mischmetalloxide besitzen große Oberflächen und Porositäten vergleichbar mit denen von Zeolithen. Sie können zur formselektiven Katalyse von Isomerisierungsreaktionen, Hydrierreaktionen, selektive und unselelektive Oxidationsreaktionen mit Luftsauerstoff, Wasserstoffperoxid oder organischen Peroxiden, Alkylierungsreaktionen, Disproportinierungsreaktionen, Hydrier- und Dehydrierreaktionen, Alkoholbildung aus Olefinen, Kupplungsreaktionen, Substitutionsreaktionen, Cycloadditions- oder Cycloreversionsreaktionen, Etherbildung, Rohölcracking und Hydrocracking, Fischer-Tropsch-Synthese von Alkoholen oder Kohlenwasserstoffen, Methanolsynthese aus Synthesegas oder aus Methan eingesetzt werden. Sie können zur Beschichtung von Elektroden in Brennstoffzellen oder Li+ oder anderen Ionenspeichern in Batterien Einsatz finden. Sie können auch als Ionenaustauscher oder als Adsorptionsmittel Verwendung finden. Sie eignen sich auch für die Bildung von Ultrafiltration und Gastrennmembranen (DE - A - 41 17 284), für die Bildung von Katalysatoren mit selektiven Hohlräumen zur molekularen Erkennung (DE - A -43 09 660).

Die Porengröße dieser mikroporösen Gläser kann zwischen 0,4 - 1 nm durch Änderung der Alkoholgröße in Polykondensationsprozessen oder durch verändertes Trocknungs- und Kalzinierverfahren variiert werden. Die Hydrophobizität der inneren Oberfläche dieser Materialien kann durch Cokondensation von Alkylmetallverbindungen, vorzugsweise Alkyltrialkoxysilanen während des Sol-Gel-Prozesses maßgeschneidert werden.

Herstellung der Titandioxid-Siliziumdioxid-Gläser mit unterschiedlicher Titan-Verbindung:

### Beispiel 1

10 ml Tetraethoxysilan (TEOS), 0,14 ml (EtO)₄Ti und 8 ml Ethanol werden hintereinander ineinander gelöst und 1,6 ml 8 n HCl unter Rühren zugegeben. Nach erfolgter Gel-Bildung wird das Material auf 65 °C mit einer Heizrate von 0,5 °C/min aufgeheizt, 3 h bei 65 °C gehalten, mit einer Heizrate von 0,2 °C/min auf 250 °C aufgeheizt und weitere 3 h bei dieser Temperatur kalziniert. Die Ad- Desorptionsisothermen zeigen, daß das Material eine echte monomodale Mikroporenverteilung besitzt, BET: 471 m²/g, Porendurchmesser 0,67 nm.

### Beispiel 2

### Andere Titantetraalkoholate sind brauchbar.

46,3-x mmol TEOS, x mmol (iPrO)₄Ti, wobei x = 0 bis 43,3 mmol betragen kann, und 140 mmol Ethanol werden hintereinander ineinander gelöst und 2 ml 8 n HCl unter Rühren zugegeben. Die Mischung wird 3 Tage bei Raumtemperatur gehalten. Nach erfolgter Gel-Bildung wird das Material auf 65 °C mit einer Heizrate von 0,5 °C/min aufgeheizt, 3 h bei 65 °C gehalten, mit einer Heizrate von 0,2 °C/min auf 250 °C aufgeheizt und weitere 3 h bei dieser Temperatur kalziniert. Die Ad- Desorptionsisothermen zeigen, daß das Material eine echte monomodale Mikroporenverteilung besitzt, BET: 180 (60 % Ti) bis 600 (3 % Ti) m²/g, Porendurchmesser 0,73-0,8 nm.

### Beispiel 3

### Vanadinoxid-Siliziumdioxid-Glas

10 ml Tetraethoxysilan (TEOS), 1,2 g Vanadylacetylacetonat (O=V(AcAc)₂ und 8 ml Ethanol werden hintereinander ineinander gelöst und 2 ml 8 n HCl unter Rühren zugegeben. Nach erfolgter Gel-Bildung wird das Material mit einer Heizrate von 0,5 °C/min auf 65 °C aufgeheizt, 3 h bei 65 °C gehalten, mit einer Heizrate von 0,2 °C/min auf 250 °C aufgeheizt und weitere 3 h bei dieser Temperatur kalziniert. Die Ad- Desorptionsisothermen zeigen, daß das Material eine echte monomodale Mikroporenverteilung besitzt, BET: 542 m²/g, Porendurchmesser 0,66 nm.

### Beispiel 4

### Zirkondioxid-Siliziumdioxid-Glas

10 ml Tetraethoxysilan (TEOS), 1 ml (BuO)₄Zr und 8,4 ml Ethanol werden hintereinander ineinander gelöst und 2 ml 2 n HCl unter Rühren zugegeben. Nach erfolgter Gel-Bildung wird das Material auf 65 °C mit einer Heizrate von 0,5 °C/min aufgeheizt, 3 h bei 65 °C gehalten, mit einer Heizrate von 0,2 °C/min auf 250 °C aufgeheizt und weitere 3 h bei dieser Temperatur kalziniert. Die Ad- Desorptionsisothermen zeigen, daß das Material eine echte monomodale Mikroporenverteilung besitzt, BET: 240 m²/g, Porendurchmesser 0,65 nm.

### Beispiel 5

### Aluminiumoxid-Siliziumdioxid-Glas

27,5 ml (EtO)₄Si, 0,45 ml Triisobutylaluminium, 2,01 ml tetra-n-Propoxyzirkonium und 25 ml Ethanol werden hintereinander ineinander gelöst und 4,5 ml 0,4 n HCl unter Rühren während 10 min zugegeben. Dabei steigt die Temperatur bis auf 60 °C. Nach erfolgter Gel-Bildung und langsamer Vortrocknung bei Raumtemperatur wird das Material auf 65 °C mit einer Heizrate von 0,5 °C/min aufgeheizt, 3 h bei 65 °C gehalten, mit einer Heizrate von 0,2 °C/min auf 300 °C aufgeheizt und weitere 3 h bei dieser Temperatur kalziniert. Die Ad- Desorptionsisothermen zeigen, daß das Material eine echte monomodale Mikroporenverteilung besitzt, BET: 280 m²/g, Porendurchmesser 0,7 nm.

### Beispiel 6

### Titandioxid-Zirkondioxid-Siliziumdioxid-Glas

10 ml Tetraethoxysilan (TEOS), 1 ml (BuO)₄Zr und 8 ml Ethanol werden hintereinander ineinander gelöst und 2 ml 8 n HCl unter Rühren zugegeben. Nach erfolgter Gel-Bildung wird das Material auf 65 °C mit einer Heizrate von 0,5 °C/min aufgeheizt, 3 h bei 65 °C gehalten, mit einer Heizrate von 0,2 °C/min auf 250 °C aufgeheizt und weitere 3 h bei dieser Temperatur kalziniert. Die Ad- Desorptionsisothermen zeigen, daß das Material eine echte monomodale Mikroporenverteilung besitzt, BET: 520 m²/g, Porendurchmesser 0,75 nm.

### Beispiel 7

### Formselektivität der Epoxidation von Alkenen

15,8 mmol Alken, 1 ml 3 n Lösung t-Butylhydroperoxid und 50 mg Titanoxid-Siliziumoxid-Glas werden bei 80 °C 15 min gerührt und die Zusammensetzung des Produktes mit Hilfe der Gaschromatographie analysiert. Die relative Umwandlung der einzelnen Alkene in Epoxide bezogen auf die langsamste Reaktion gleich 1 wird in Klammern angegeben: 1-Hexen (9), 1-Hepten (3), 1-Octen (4), 1-Nonen (3), 1-Decen (3), 1-Dodecen (2), 1-Pentadecen (1), Cyclohexen (9), Cyclohepten (2), Cycloocten (1) und Cyclodecen (1). Da die verschiedenen endständigen und die verschiedenen Cyclo-Alkene in etwa die gleiche Reaktivität unter homogenen Bedingungen der Epoxibildung haben, weisen die Resultate auf eine starke Bevorzugung der Epoxibildung der kleinen Alkene hin, was auf eine molekulare Formselektivität weist, verursacht durch monomodale Mikroporen des Glases.

### Beispiel 8

### Formselektivität zur Bildung von t-Butylether

In einem 100 ml Autoklav werden 300 mmol i-Buten, 100 mmol 1-Hexanol und 2,5 g Ti-Si-Katalysatorpulver bei einem Druck von 40 bar N₂ und einer Temperatur von 150 °C 18 h gerührt. Es wurde eine Umformung von 62 % des n-Hexanol unter Bildung von Hexyl-t-buthylether mit einer Selektivität von 92 % erzielt.

### Beispiel 9

### Formselektivität zur Bildung von t-Butylether

In einem 100 ml Autoklav werden 300 mmol t-Butanol, 100 mmol 1-Hexanol und 2,5 g Ti-Si-Katalysatorpulver bei einem Druck von 40 bar N₂ und einer Temperatur von 150 °C 17 h gerührt. Es wurde eine Umformung von 17 % des n-Hexanol unter Bildung von Hexyl-t-buthylether mit einer Selektivität von 90 % erzielt.

### Beispiel 10

### Formselektivität beim Hydrocracking von n-Decan.

Ein mikroporöses amorphes TiSi-Mischoxid-Glas wurde mit 1 % Pt impregniert und dem standardisierten Hydrocracktest wie in der Literatur beschrieben (J.A. Martens, M. Tielen, P.A. Jacobs, J. Weitkamp, Zeolites 4 (1984) 98-107) ausgesetzt. Das mikroporöse Ti-Si-Glas zeigte einen Constraint Index (CI∗) von 1,5, einen EC8 (% Ethyloctan) von 10,3, einen PC7 (% 4-Propylheptan) von 0,9, einen DB iC10 (% doppeltverzweigte C10-Isomere) von 30, einen iC5 (Isopentane im Crackprodukt) von 30,5, und einen Porendimensions-Index DI von 14,9. Der CI^{∗} kann zur Porengrößenabschätzung herangezogen werden und zeigt, daß die Poren größer sind als im ZSM12 und kleiner als im Y-Zeolithen. Der EC8 bestätigt, daß die Bronsted-sauren Zentren in den Poren lokalisiert sind und daß sich das Material ähnlich wie ein SAPO-5-Zeolith verhält. PC7 zeigt, daß die Poren ähnlich den Poren eines FAU-Zeolithen sind. Der DBiC10 ist ähnlich dem von SAPO-5, was auf ein ähnliches Porensystem weist. Der iC5 bestätigt, daß das Glas zu den großporigen Zeolithen gehört. Der DI weist auf die Abwesenheit von verbundenen Poren. Insgesamt zeigt der Hydrocrack-Test von Dekan, daß die Broensted-sauren Zentren des Glases sich in einer formselektiven Umgebung befinden. Die Mikroporen des Glases haben eine röhrenartige Form mit einem Porendurchmesser von 0,7-0,8 nm.

### Beispiel 11

Herstellung eines 2 mol% In in Si Mischoxidkatalysators: 0,471 g Indiumnitrat wurden in einem 50 ml PP-Becher in 14 ml Ethanol unter Rühren gelöst. Nach der langsamen Zugabe von 20 ml TEOS wurden 3,49 ml 8 N HCl zugetropft. Die klare Lösung wurde dann weiter für mehrere Tage gerührt, bis Gelierung eintrat. Das transparente Glas wurde dann in kleinere Stücke gebrochen und an der Luft bei 250 °C für 10 h kalziniert (Aufheizgeschwindigkeit 2,5 °C/min). Nach der Kalzinierung wurden die Katalysatorteilchen auf eine Partikelgröße < 200 µm gemahlen.

### Beispiel 12

Selektive Oxidation von Propen mit Luft zu 1,5-Hexadien: 50 mg des 2 % In in Si Mischoxidkatalysators (Beispiel 11) wurden in der Mitte des Quartzrohrs eines Gasphasenströmungsreaktors (Länge 30 cm, innerer Durchmesser 4 mm) dicht gepackt und auf die Reaktionstemperatur von 590 °C erhitzt, während die Gasmischung aus 90 vol% Propen und 10 % Luft (Strömungsgeschw.(Propen) = 37,8 ml/min; (Luft) = 4,2 ml/min) kontinuierlich durch das Katalysatorbett strömte. Nachdem sich ein stationärer Zustand eingestellt hatte, wurde die Produktgasmischung in einer Kühlfalle bei -78 °C ausgefroren und gaschromatographisch analysiert. Es ergab sich bei einem Propenumsatz von 5-10 % eine 1,5-Hexadienselektivität von 96 %.

### Beispiel 13

Herstellung eines 6 mol% Cu in Si Mischoxidkatalysators: 1,313 g Kupfer(II)-diacetylacetonat (Cu(acac)₂) wurden in einem 50 ml PP-Becher in 14,6 ml Ethanol unter Rühren gelöst. Nach der langsamen Zugabe von 20 ml TEOS wurden 3,63 ml 8 N HCl zugetropft. Die klare Lösung wurde dann weiter für mehrere Tage gerührt, bis Gelierung eintrat. Das transparente Glas wurde dann in kleinere Stücke gebrochen und an der Luft bei 250 °C für 10 h kalziniert (Aufheizgeschwindigkeit 2,5 °C/min). Nach der Kalzinierung wurden die Katalysatorteilchen auf eine Partikelgröße < 200 µm gemahlen.

### Beispiel 14

Selektive Oxidation von Propen zu Acrolein mit Luft: 50 mg des 6 % Cu in Si Mischoxidkatalysators (Beispiel 13) wurden in der Mitte des Quartzrohrs des Reaktors dicht gepackt (identischer Reaktor wie in Beispiel 12) und auf die Reaktionstemperatur von 370 °C erhitzt, während die Gasmischung aus 28,6 vol% Propen und 71,4 % Luft (Strömungsgeschw.(Propen) = 25 ml/min; (Luft) = 62,5 ml/min) kontinuierlich durch das Katalysatorbett strömte. Nachdem sich ein stationärer Zustand eingestellt hatte, wurde die Produktgasmischung in einer Kühlfalle bei -78 °C ausgefroren und gaschromatographisch analysiert. Es ergab sich bei einem Propenumsatz von 4-8 % eine Acroleinselektivität von 85 %.

## Patentansprüche

1. Verfahren zur Herstellung formselektiv katalytisch aktiver, amorpher, mikroporöser Mischmetalloxide nach dem Sol-Gel-Verfahren, dadurch gekennzeichnet, daß mindestens zwei hydrolysierbare, flüssige oder gelöste Verbindungen der Elemente Titan, Silicium, Aluminium, Zirkon oder Cer hintereinander ineinander gelöst werden, die klare Lösung bei pH 0 bis 7 unter Zusatz wässriger saurer Katalysatoren oder unter Zusatz von Fluoridionen unter linearer Polymerisation oder Polykondensation gerührt werden, das erhaltene Gel durch Erwärmen auf 60 bis 70 °C schonend getrocknet wird und mit kleinen Aufheizgeschwindigkeiten bei Temperaturen von 120 bis 800 °C kalziniert wird, wobei ein mikroporöses, amorphes Glas erhalten wird.

2. Verfahren nach Anspruch 1, dadurch gekennzeichnet, daß die erhaltenen mikroporösen, amorphen, nicht-keramischen Gläser aus einer Mischmetalloxidmatrix bestehen, in der wenigstens etwa 90 % der Poren des Materials einen effektiven Durchmesser zwischen 0,3 und 1,2 nm haben, mit im wesentlichen gleicher Porengröße und mit einer Oberflächengröße von über 50 m²/g.

3. Verfahren nach Anspruch 1, dadurch gekennzeichnet, daß die hydrolisierbaren flüssigen oder gelösten Verbindungen aus der Gruppe SiO₂, TiO₂, Al₂O₃, Zirconiumoxid, Ceriumoxid, Spinell, Mullit, Siliciumcar-bid, Siliciumnitrit und Titannitrit ausgewählt sind.

4. Verfahren nach Ansprüchen 1 bis 3, dadurch gekennzeichnet, daß die Mischmetalloxidmatrix wenigstens 50 Gew.-% wenigstens einer Verbindung nach Anspruch 1 oder 3 und bis zu 50 Gew.-% eines oder mehrerer Metalloxide in atomarer Verteilung aus der Gruppe der Metalle Mo, Sn, Zn, V, Mn, Fe, Co, Ni, As, Pb, Sb; Bi, Ru, Re, Cr, W, Nb, Hf, La, Ce, Gd, Ga, In, Tl, Ag, Cu, Li, K, Na, Be, Mg, Ca, Sr und Ba enthält.

5. Verfahren nach Ansprüchen 1 bis 4, dadurch gekennzeichnet, daß die Mischmetalloxidmatrix zusätzlich bis zu 5 Gew.-% wenigstens eines der Edelmetalle Pt, Rh, Ir, Os, Ag, Au, Cu, Ni, Pd, Co in hochdisperser Form in metallischem oder oxidiertem Zustand enthält.

6. Verfahren nach Ansprüchen 1 bis 5, dadurch gekennzeichnet, daß die sauren Katalysatoren Säuren sind.

7. Verfahren nach Anspruch 6, dadurch gekennzeichnet, daß die Säure Salzsäure ist.

8. Verfahren nach Ansprüchen 1 bis 7, dadurch gekennzeichnet, daß die Kalzinierungstemperatur 250 bis 500 °C beträgt.

9. Verfahren nach Ansprüchen 1 - 4 und 6 - 8, dadurch gekennzeichnet, daß die hydrolysierbaren, löslichen Verbindungen reine Alkoxy-, gemischte Alkoxy-, Alkoxyoxooder Acetylacetonat-Derivate der gewählten Metalle oder Metalloxide sind.

10. Mikroporöse amorphe, nicht-keramische Gläser, bestehend aus einer Mischmetalloxidmatrix, in der wenigstens etwa 90 % der Poren des Materials einen effektiven Durchmesser zwischen 0,3 und 1,2 nm haben, mit im wesentlichen gleicher Porengröße und mit einer Oberflächengröße von über 50 m₂/g.

11. Mikroporöse, amorphe nicht-keramische Gläser nach Anspruch 10, dadurch gekennzeichnet, daß die Mischmetalloxidmatrix aus wenigstens zwei der Oxide von Titan, Silicium, Aluminium, Zirkon oder Cer besteht.

12. Mikroporöse, amorphe, nicht-keramische Gläser nach Anspruch 10 und 11, dadurch gekennzeichnet, daß die Mischmetalloxidmatrix aus wenigstens zwei der Verbindungen aus der Gruppe SiO₂, TiO₂, Al₂O₃, Zirconiumoxid, Ceriumoxid, Spinell, Mullit, Siliciumcarbid, Siliciumnitrit und Titannitrit besteht.

13. Mikroporöse, amorphe, nicht-keramische Gläser, dadurch gekennzeichnet, daß die Mischmetalloxidmatrix aus wenigstens 50 Gew.-% einer der Verbindungen nach Ansprüchen 11 und 12 und bis zu 50 Gew.-% einer oder mehrerer Metalloxide in atomarer Verteilung aus der Gruppe der Metalle Mo, Sn, Zn, V, Mn, Fe, Co, Ni, As, Pb, Sb, Bi, Ru, Re, Cr, W, Nb, Hf, La, Ce, Gd, Ga, In, Tl, Ag, Cu, Li, K, Na, Be, Mg, Ca, Sr und Ba besteht.

14. Mikroporöse, amorphe, nicht-keramische Gläser nach Ansprüchen 10 bis 13, zusätzlich enthaltend bis zu 5 Gew.-% wenigstens eines der Edelmetalle Pt, Rh, Ir, Os, Ag, Au, Cu, Ni, Pd, Co in hoch-disperser Form in metallischem oder oxidiertem Zustand.

15. Mikroporöse, amorphe, nicht-keramische Gläser nach Ansprüchen 10 bis 14, erhalten durch saure oder Fluorid-katalysierte lineare Polymerisation oder Polykondensation hydrolisierbarer löslicher Verbindungen gemäss Ansprüchen 11 bis 13, vorzugsweise reine Alkoxy-, gemischte Alkoxy-, Alkoxyoxo- oder Acetylacetonat-Derivate der gewählten Metalle oder Metalloxide bei pH 0 bis 7 in einem Sol-Gel-Verfahren, gefolgt von mildem Trocknen und langsamem Kalzinieren mit einer Endkalzininierungstemperatur im Bereich von 120 bis 800 °C, wobei die mikroporösen, amorphen, nicht-keramischen Gläser entweder ausschließlich aus den gemischten Metalloxiden oder aus den gemischten Metalloxiden und restlichen Oberflächenalkyl- oder - alkoxygruppen in Abhängigkeit von der gewählten Vorläufer-Verbindung bestehen.

16. Formselektive Katalysatoren, bestehend aus mikroporösen, amorphen, nicht-keramischen, Mischmetalloxidgläsern nach Ansprüchen 10 bis 15.

17. Verwendung von mikroporösen, amorphen, nicht-keramischen Mischmetalloxidgläsern bzw. formselektiven Katalysatoren zur Katalyse von Isomerisierungsreaktionen, Hydrierreaktionen, selektiven und unselektiven Oxidationsreaktionen mit Luftsauerstoff, Wasserstoffperoxid oder organischen Peroxiden, Alkylierungsreaktionen, Disproportionierungsreaktionen, Hydrier- und Dehydrierreaktionen, Alkoholbildung aus Olefinen, Kupplungsreaktionen, Substitutionsreaktionen, Cycloadditions- oder Cycloreversionsreaktionen, Etherbildung, Rohölcracking und Hydrocracking, Fischer-Tropsch-Synthese von Alkoholen oder Kohlenwasserstoffen, Methanolsynthese aus Synthesegas oder aus Methan, zur Beschichtung von Elektroden in Brennstoffzellen oder Li⁺ oder anderen Ionenspeichern in Batterien, und zur Bildung von Ultrafiltration- und Gastrennmembranen, und zur Bildung von Katalysatoren mit selektiven Hohlräumen zur molekularen Erkennung.

## Claims

1. A process for the preparation of shape selective catalytic active, amorphous, microporous mixed metal oxides according to the sol-gel process, characterized in that at least two hydrolyzable, liquid or dissolved compounds of the elements titanium, silicon, aluminium, zirconium or cerium are dissolved successively in each other, the clear solution is stirred at a pH of from 0 to 7 while fluoride ions are added to effect linear polymerization or polycondensation, the obtained gel is dried under mild conditions by heating at 60 to 70°C, and is calcinated at temperatures in the range of from 120 to 800°C with slow heating rates, whereby a microporous, amorphous glass is obtained.

2. The process according to claim 1, characterized in that the obtained microporous, amorphous, non-ceramic glasses consist of a matrix of mixed metal oxides, in which at least about 90% of the pores of the material have an effective diameter of between 0.3 and 1.2 nm, principally the same pore size, and a surface area of more than 50 m²/g.

3. The process according to claim 1, characterized in that the hydrolyzable liquid or dissolved compounds are selected from the group consisting of SiO₂, TiO₂, Al₂O₃, zirconium oxide, cerium oxide, spinel, mullite, silicon carbide, silicon nitride and titanium nitride.

4. The process according to the claims 1 to 3, characterized in that the matrix of mixed metal oxides contains at least 50% by weight of a compound according to claim 1 or 3 and up to 50% per weight of one or more metal oxides in an atomic distribution, selected from the group of metals consisting of Mo, Sn, Zn, V, Mn, Fr, Co, Ni, As, Pb, Sb, Bi, Ru, Re, Cr, W, Nb, Hf, La, Ce, Gd, Ga, In, Tl, Ag, Cu, Li, K, Na, Be, Mg, Ca, Sr and Ba.

5. The process according to the claims 1 to 4, characterized in that the matrix of mixed metal oxides additionally contains up to 5% per weight of at least one of the noble metals Pt, Rh, Ir, Os, Ag, Au, Cu, Ni, Pd, Co in a highly dispersed form in a metallic or oxidized state.

6. The process according to the claims 1 to 5, characterized in that the acidic catalysts are acids.

7. The process according to claim 6, characterized in that the acidic catalyst is hydrochloric acid.

8. The process according to the claims 1 to 7, characterized in that the calcination temperature is from 250 to 500°C.

9. The process according to the claims 1 to 4 and 6-8, characterized in that the hydrolyzable, soluble compounds are pure alkoxy, mixed alkoxy, alkoxyoxo or acetylacetonate derivatives of the selected metals or metal oxides.

10. The microporous, amorphous, non-ceramic glasses, consisting of a matrix of mixed metal oxides, in which at least about 90% of the pores of the material have an effective diameter between 0.3 and 1.2 nm, with substantially equal pore sizes, and have a surface area of more than 50 m²/g.

11. The microporous, amorphous, non-ceramic glasses according to claim 10, characterized in that the matrix of mixed metal oxides consists of at least two oxides selected from the oxides of titanium, silicon, aluminium, zirconium or cerium.

12. The microporous, amorphous, non-ceramic glasses according to claim 10 or 11, characterized in that the matrix of mixed metal oxides consists of at least two compounds selected from the group consisting of SiO₂, TiO₂, zirconium oxide, cerium oxide, spinel, mullite, silicon carbide, silicon nitride and titanium nitride.

13. The microporous, amorphous, non-ceramic glasses, characterized in that the matrix of mixed metal oxides consists of at least 50% by weight of one of the compounds according to claim 11 or 12, and up to 50 % per weight of one or more metal oxides in an atomic distribution selected from the group of metals consisting of Mo, Sn, Zn, V, Mn, Fr, Co, Ni, As, Pb, Sb, Bi, Ru, Re, Cr, W, Nb, Hf, La, Ce, Gd, Ga, In, Tl, Ag, Cu, Li, K, Na, Be, Mg, Ca, Sr and Ba.

14. The microporous, amorphous, non-ceramic glasses according to the claims 10 to 13, additionally containing up to 5% by weight of at least one of the noble metals Pt, Rh, Ir, Os, Ag, Au, Cu, Ni, Pd, Co in a highly dispersed form in a metallic or oxidized state.

15. The microporous, amorphous, non-ceramic glasses according to the claims 10 to 14, obtained by acid or fluoride catalyzed linear polymerization or polycondensation of hydrolyzable, soluble compounds according to the claims 11 to 13, preferably pure alkoxy, mixed alkoxy, alkoxyoxo or acetylacetonate derivatives of the selected metals or metal oxides at a pH of 0 to 7 in a sol-gel process, followed by mild drying and slow calcination with a final calcination temperature in the range of 120 to 800°C, whereby the resulting microporous, amorphous, non-ceramic glasses consist either of the mixed metal oxides or the mixed metal oxides and the residual surface alkyl or alkoxy groups dependent on the selected precursor compound.

16. Shape selective catalysts consisting of microporous, amorphous, non-ceramic glasses of mixed metal oxides according to the claims 10 to 15.

17. The use of microporous, amorphous, non-ceramic glasses of mixed metal oxides or shape selective catalysts for the catalysis of isomerization reactions, hydrogenation reactions, selective and non-selective oxidation reactions with atmospheric oxygen, hydrogen peroxide or organic peroxides, alkylation reactions, disproportionation reactions, hydrogenation and dehydrogenation reactions, formation of alcohol from olefins, coupling reactions, substitution reactions, cycloaddition reactions or cyclo-reversion reactions, ether formation, cracking of crude oil and hydrocracking, Fischer-Tropschsynthesis of alcohols or hydrocarbons, synthesis of methanol from synthesis gas or methane, for the coating of electrodes in fuel cells or Li⁺ or other ion storages in batteries, and for the preparation of membranes for ultrafiltration and gas separation, and for the preparation of catalysts with selective cavities for molecular identification.

## Revendications

1. Procédé de préparation d'oxydes métalliques mixtes microporeux amorphes catalytiquement actifs avec sélection par la forme selon le procédé sol-gel, caractérisé en ce qu'on dissout l'un avec l'autre et successivement au moins deux composés hydrolysables, liquides ou dissous, des éléments titane, silicium, aluminium, zirconium ou cérium, en ce qu'on agite la solution claire à pH compris entre 0 et 7 avec addition de catalyseurs acides aqueux ou avec addition d'ions fluorure avec polymérisation linéaire ou polycondensation, en ce qu'on sèche de façon douce le gel obtenu en le chauffant à 60 jusqu'à 70°C et en ce qu'on le calcine à de faibles vitesses de chauffage à des températures de 120 à 800°C, opération qui fournit un gel amorphe microporeux.

2. Procédé selon la revendication 1, caractérisé en ce que les verres non céramiques amorphes microporeux obtenus se composent d'une matrice d'oxyde métallique mixte dans laquelle au moins environ 90% des pores du matériau ont un diamètre effectif compris entre 0,3 et 1,2 nm, avec une taille de pores essentiellement régulière et une surface supérieure à 50 m²/g.

3. Procédé selon la revendication 1, caractérisé en ce que les composés liquides ou dissous hydrolysables sont choisis dans le groupe constitué par SiO₂, TiO₂, Al₂O₃, l'oxyde de zirconium, l'oxyde de cérium, les spinelles, la mullite, le carbure de silicium, le nitrure de silicium et le nitrure de titane.

4. Procédé selon les revendications 1 à 3, caractérisé en ce que la matrice d'oxyde métallique mixte contient au moins 50% en poids d'un composé selon la revendication 1 ou 3 et jusqu'à 50% en poids d'un ou plusieurs oxydes métalliques en répartition atomique du groupe des métaux Mo, Sn, Zn, V, Mn, Fe, Co, Ni As, Pb, Sb; Bi, Ru, Re, Cr, W, Nb, Hf, La, Ce, Gd, Ga, In, Tl, Ag, Cu, Li, K, Na, Be, Mg, Ca, Sr et Ba.

5. Procédé selon les revendications 1 à 4, caractérisé en ce que la matrice d'oxyde métallique mixte contient en outre jusqu'à 5% en poids d'au moins un des métaux précieux Pt, Rh, Ir, Os, Ag, Au, Cu, Ni, Pd, Co sous forme hautement dispersée à l'état métallique ou oxydé.

6. Procédé selon les revendications 1 à 5, caractérisé en ce que les catalyseurs acides sont des acides.

7. Procédé selon la revendication 6, caractérisé en ce que l'acide est l'acide chlorhydrique.

8. Procédé selon les revendications 1 à 7, caractérisé en ce que la température de calcination s'élève à 250 à 500°C.

9. Procédé selon les revendications 1 - 4 et 6 - 8 , caractérisé en ce que les composés solubles hydrolysables sont des dérivés purs d'alcoxy, d'alcoxy mixtes, d'alcoxyoxo ou d'acétylacétonate des métaux ou oxydes métalliques choisis.

10. Verres non céramiques amorphes microporeux constitués d'une matrice d'oxyde métallique mixte dans laquelle au moins environ 90% des pores du matériau ont un diamètre effectif compris entre 0,3 et 1,2 nm, avec une taille de pores essentiellement identique et avec une surface supérieure à 50 m²/g.

11. Verres non céramiques amorphes microporeux selon la revendication 10, caractérisés en ce que la matrice d'oxyde métallique mixte se compose d'au moins deux des oxydes de titane, de silicium, d'aluminium, de zirconium ou de cérium.

12. Verres non céramiques amorphes microporeux selon les revendications 10 et 11, caractérisés en ce que la matrice d'oxyde métallique mixte se compose d'au moins deux des composés du groupe SiO₂, TiO₂, Al₂O₃, oxyde de zirconium, oxyde de cérium, spinelles, mullite, carbure de silicium, nitrure de silicium et nitrure de titane.

13. Verres non céramiques amorphes microporeux, caractérisés en ce la matrice d'oxyde métallique mixte se compose d'au moins 50% en poids d'un des composés selon les revendications 11 et 12 et de jusqu'à 50% en poids d'un ou plusieurs oxydes métalliques en répartition atomique du groupe des métaux Mo, Sn, Zn, V, Mn, Fe, Co, Ni, As, Pb, Sb, Bi, Ru, Re, Cr, W, Nb, Hf, La, Ce, Gd, Ga, In, Tl, Ag, Cu, Li, K, Na, Be, Mg, Ca, Sr et Ba.

14. Verres non céramiques amorphes microporeux selon les revendications 10 à 13, contenant en outre jusqu'à 5% en poids d'au moins un des métaux précieux Pt, Rh, Ir, Os, Ag, Au, Cu, Ni, Pd, Co sous forme finement dispersée à l'état métallique ou oxydé.

15. Verres non céramiques amorphes microporeux selon les revendications 10 à 14, obtenus par une polymérisation ou polycondensation linéaire, à catalyse acide ou par un fluorure, de composés solubles hydrolysables selon les revendications 11 à 13, de préférence de dérivés alcoxy purs, de dérivés alcoxy mixtes, de dérivés alcoxyoxo ou de dérivés acétylacétonate des métaux ou oxydes métalliques choisis à un pH compris entre 0 et 7 dans un procédé sol-gel, suivi par un séchage modéré et une lente calcination avec une température de calcination finale comprise entre 120 et 800°C, où les verres non céramiques amorphes microporeux se composent ou bien exclusivement des oxydes métalliques mixtes, ou bien des oxydes métalliques mixtes et des groupes alky]e ou alcoxy superficiels résiduels en fonction du composé précurseur choisi.

16. Catalyseurs à sélection par la forme, constitués de verres d'oxydes métalliques mixtes non céramiques amorphes microporeux selon les revendications 10 à 15.

17. Utilisation de verres d'oxydes métalliques mixtes non céramiques, amorphes et microporeux ou selon les cas de catalyseurs à sélection par la forme, pour la catalyse de réactions d'isomérisation, de réactions d'hydrogénation, de réactions d'oxydation sélective et non sélective avec l'oxygène de l'air, du peroxyde d'hydrogène ou des peroxydes organiques, de réactions d'alkylation, de réactions de dismutation, de réactions d'hydrogénation et de déshydrogénation, de formation d'alcool à partir d'oléfines, de réactions de couplage, de réactions de substitution, de réactions de cycloaddition ou de cycloréversion, de formation d'éthers, de craquage et d'hydrocraquage de pétrole brut, de synthèse de Fischer-Tropsch d'alcools ou d'hydrocarbures, de synthèse de méthanol à partir de gaz de synthèse ou de méthane, de revêtement d'électrodes dans des piles à combustibles ou d'accumulateur de Li⁺ ou d'autres accumulateurs d'ions dans des piles, et pour la formation de membranes d'ultrafiltration et de séparation de gaz, ainsi que pour la formation de catalyseurs comportant des espaces creux sélectifs pour la reconnaissance moléculaire.
